# EUROPÄISCHE PATENTANMELDUNG

(11) **EP 4 201 950 A1**
(43) Veröffentlichungstag der Anmeldung: **28.06.2023**
(21) Anmeldenummer: 21217028.6
(22) Anmeldetag: 22.12.2021
(51) Int. Cl.: C07J 71/00, A61P 35/00, C07J 9/00

(54) **EPOXYSTEROIDE**

(71) Anmelder: Helios Kliniken GmbH, 10117 Berlin (DE)
(72) Erfinder: Schmalz, Hans-Günther, 50321 Brühl (DE); Wilczek, Tobias, 51469 Bergisch Gladbach (DE); Taspinar, Ömer, 50735 Köln (DE); Prokop, Aram, 19063 Schwerin (DE); Baas, Jennifer, 21465 Wentorf bei Hamburg (DE)
(74) Vertreter: dompatent von Kreisler Selting Werner - Partnerschaft von Patent- und Rechtsanwälten mbB

(57) **Zusammenfassung**

Arzneimittel auf Basis von 7,19-epoxy-Steroiden, enthaltend eine Verbindung der allgemeinen Formel A 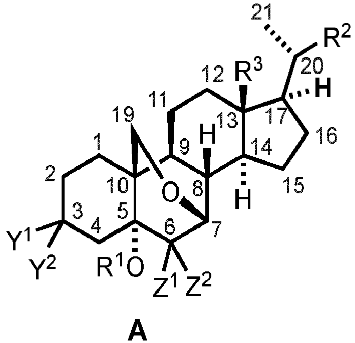 oder ein physiologisch verträgliches Salz hiervon, worin in der Formel bedeuten:
R¹ = H; Methyl, Aryl-CH₂-, R^{x}-C(=O)- oder R^{x}-O-C(=O)- ; oder R¹O entfällt, wenn eine Doppelbindung zwischen 4-5 vorhanden ist;
R² = gesättigte oder ungesättigte, gegebenenfalls verzweigte Alkylreste mit 4 bis 9 C-Atomen, wobei diese Alkylreste können auch eine Cyclopropan-oder Cyclobutan-Substruktur aufweisen;
R³= Methyl oder Ethyl,
Y¹, Y² = (H/OR⁵), oder zusammen O, NOR⁴;
Z¹, Z² = (H/OR⁵), oder zusammen O, NOR⁴;
R⁴ unabhängig voneinander = H, Alkyl oder Aryl; und
R⁵ unabhängig voneinander = H; Alkyl, Aryl-CH₂-, R^{x}-C(=O)- oder R^{x}-O-C(=O)-;
wobei jedes R^{x} unabhängig voneinander = H, gesättigte oder ungesättigte, gegebenenfalls verzweigtes Alkyl, gesättigte oder ungesättigte, gegebenenfalls verzweigtes substituiertes Alkyl, Aryl, Heteroaryl, PEG, SO₃H, PO(OH)₂ oder Glycosyl ist;
und wobei bis zu 3 Wasserstoffe durch Fluor substituiert sein können.

## Beschreibung

Die vorliegende Erfindung betrifft neue Arzneimittel auf Basis von Steroiden.

Die Entstehung von malignen Tumoren und Leukämien bzw. anderen gutartigen hyperproliferativen Erkrankungen, wie z. B. der Schuppenflechte oder des Keloids, ist auf eine gestörte Balance zwischen der Gewebsneubildung und dem regulierten Absterben von Zellen aus dem Gewebsverband zurückzuführen.

In der klinischen Praxis wird durch den Einsatz zytotoxischer Behandlungsmethoden, wie z. B. der Chemotherapie, der Strahlentherapie und der Hyperthermie, versucht, diese gestörte Balance wieder zurechtzurücken und die überschüssigen Tumorzellen gleichzeitig abzutöten. Es ist allgemein anerkannt, dass die meisten derzeit in der klinischen Praxis eingesetzten Chemotherapeutika ihre Wirkung durch Einleitung der Apoptose (programmierter Zelltod) entfalten (Hannun, 1997). Allerdings entwickelt ein Teil der an malignen Tumoren erkrankten Patienten frühzeitig eine Chemotherapie- bzw. Strahlenresistenz oder ist primär therapierefraktär (Hickman, 1996). Weiterhin ist bekannt, dass Primärtumor und Metastasen auf zytotoxische Therapien oft ganz different ansprechen.

Aufgrund neuerer Untersuchungen ist bekannt, dass die Ursachen für Resistenzen oft in unterschiedlichen Störungen der Apoptose-Signalkaskade liegen (Raisova et al., 2000).

Insbesondere die Therapien von Rezidiven bei Leukämien, Lymphomen und soliden Tumoren im Kindesalter zeigen noch immer keine befriedigenden Überlebensraten. So versterben z. B. trotz aggressiver Therapie ca. 45% der erkrankten Kinder im Rezidiv der akuten lymphatischen Leukämie (ALL). Ähnliches trifft auch auf andere teilweise schwer therapierbare Tumorerkrankungen, wie z. B. Hirntumore, Lymphome, Sarkome, wie Weichteilsarkome, Ewing- oder Osteosarkom, Keimzell-Tumore, Nieren- oder Lebertumore und Karzinome, wie z. B. das Schilddrüsenkarzinom oder die im Erwachsenenalter sehr häufigen Kolonkarzinome und Mammakarzinome, zu. Ferner fehlt bei schwierigen seltenen Tumoren wie z. B. beim desmoblastischen Rundzell-Tumor oder beim malignen peripheren Nervenscheiden-Tumor im Falle einer Metastasierung eine geeignete Chemotherapie, da diese malignen Zellen Zytostatika-Resistenzen aufweisen.

Weiterhin stellt die Psoriasis eine der häufigsten Erkrankungen der Haut (zwei bis vier Prozent der Menschen leiden an ihr) dar, deren Therapie noch immer verbesserungswürdig ist.

Obwohl eine Vielzahl von Arzneimitteln mit Wirkungen durch Apoptose bekannt ist, besteht weiterhin Bedarf nach neuen Arzneimitteln, welche die Resistenzen der malignen Zellen gegen herkömmliche Zytostatika überwinden können.

Ferner besteht ein großer Bedarf an neuen zytostatischen Wirkstoffen, die in das Zentralnervensystem eindringen können, da sie ein hohes Verteilungsvolumen zeigen. Gerade zur Behandlung von verschiedenen Hirntumoren (Grad 1 - Grad 4) ist dies von großer Bedeutung, da viele herkömmlichen Zytostatika nicht ausreichend die Blut-Hirn-Schranke überwinden können, so dass bei einer Chemotherapie nicht die notwendige Wirkstoffkonzentration im Tumorgewebe erreicht werden kann. Herkömmliche Steroide können die Blut-Hirnschranke gut überwinden, sie zeigen aber in Hirntumoren, anderen soliden Tumoren oder in Steroid-resistenten Leukämien und Lymphomen keine oder keine ausreichende zytostatische Wirkung.

Aufgabe der vorliegenden Erfindung ist es, Arzneimittel bereitzustellen, die diese Nachteile des Standes der Technik überwinden.

Gelöst wird die Aufgabe durch Arzneimittel auf Basis von 7,19-epoxy-Steroiden, enthaltend eine Verbindung der allgemeinen Formel **A** 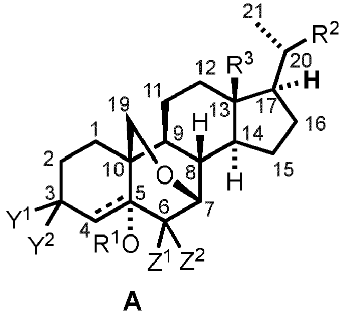 oder ein physiologisch verträgliches Salz hiervon, worin in der Formel bedeuten:
R¹ = H; Methyl, Aryl-CH₂-, R^{x}-C(=O)- oder R^{x}-O-C(=O)-, wobei R¹O entfällt, wenn eine Doppelbindung zwischen 4-5 vorhanden ist;
R² = gesättigte oder ungesättigte, gegebenenfalls verzweigte Alkylreste mit 4 bis 9 C-Atomen, wobei diese Alkylreste auch eine Cyclopropan- oder Cyclobutan-Substruktur aufweisen können;
R³= Methyl oder Ethyl;
Y¹, Y² = (H/OR⁵), oder zusammen O, NOR⁴;
Z¹, Z² = (H/OR⁵), oder zusammen O, NOR⁴;
R⁴ unabhängig voneinander = H, Alkyl oder Aryl; und
R⁵ unabhängig voneinander = H; Alkyl, Aryl-CH₂-, R^{x}-C(=O)- oder R^{x}-O-C(=O)-;
wobei jedes R^{x} unabhängig voneinander = H, gesättigte oder ungesättigte, gegebenenfalls verzweigtes Alkyl, gesättigte oder ungesättigte, gegebenenfalls verzweigtes substituiertes Alkyl, Aryl, Heteroaryl, PEG, SO₃H, PO(OH)₂ oder Glycosyl ist;
und wobei bis zu 3 Wasserstoffe durch Fluor substituiert sein können.

Die Formel A umfasst also die Grundgerüste A1 und A2:

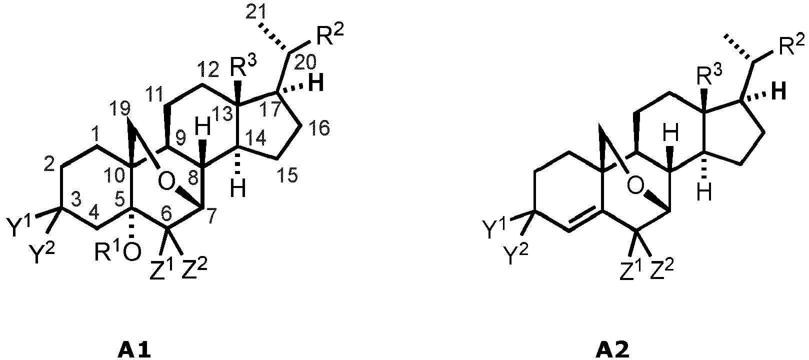

Die erfindungsgemäßen Verbindungen bauen auf dem Grundgerüst von Steroiden auf, die sowohl synthetisch als auch medizinisch/pharmakologisch gut erforscht sind.

Sie unterscheiden sich von anderen Steroiden insbesondere durch die 7,19-Epoxyverknüpfung.

Diese Substanzen leiten den apoptotischen Zelltod nicht nur in Leukämie- und Lymphomzellen sondern auch in soliden Tumorzellen und in Steroid- bzw. Polychemotherapie-resistenten Leukämie- bzw. Lymphomzellen ein. Wegen ihrer Lipophilie können diese Verbindungen der allgemeinen Formel A gegen Tumorerkrankungen des Knochenmarks, aber auch gegen Tumore einer anderen Provenienz, wie z. B. epitheliale Tumore, Sarkome oder maligne Erkrankungen der Haut usw., eingesetzt werden.

Mit den entwickelten Substanzen scheint aufgrund ihrer Lipophilie eine Überschreitung der Blut-Hirnschranke möglich und diese Substanzen könnten daher auch für maligne Hirntumore (Grad 1 - Grad 4), wie z. B. Gliome, Astrozytome, Medulloblastome oder Glioblastome, eingesetzt werden.

Aufgrund des großen Verteilungsvolumens der Steroide können vermutlich mit diesen neuen Wirkstoffen auch Tumor-Metastasen in den im menschlichen Körper für Zytostatika schwerzugänglichen Bereichen wie Pleuraspalt, Peritoneum, Mediastinum, etc. in einer zytostatischen Therapie adressiert werden.

Durch Untersuchungen der Apoptose-Signalkaskade war erfindungsgemäß die Entwicklung neuartiger Steroide möglich, die im Gegensatz zu den herkömmlichen Glukokortikoiden über alle drei bekannten Wege der Apoptose-Signalkaskade (Rezeptor-vermittelt, mitochondrial und über das endoplasmatische Retikulum vermittelt) Apoptose in bösartigen Zellen induzieren und somit Glukokortikoid- bzw. Poly-Zytostatika-resistente Leukämie-, Lymphom- und solide Tumorzellen zerstören können. Dabei ist die Wirkung selektiv auf maligne Zellen.

In einer bevorzugten Ausführungsform der Erfindung ist R¹ = H, Z¹, Z² = O, R³ = Methyl.

In einer weiteren bevorzugten Ausführungsform der Erfindung ist Y¹, Y² = (H/OH) oder Y¹, Y² = (H/OR⁵), wobei R⁵ = R^{x}-C(=O)- oder R^{x}-O-C(=O)- und R^{x} = H, Alkyl, substituiertes Alkyl, Aryl, Heteroaryl ist, wobei jeweils auch einzelne Positionen fluoriert sein können.

In einer bevorzugten Ausführungsform der Erfindung ist R^{x} = H, Alkyl und R² 4-Methyl-pentyl.

Bevorzugte Substituenten für R¹ sind H oder Methyl.

Bevorzugt weist jedes R⁴, R⁵ und R^{x} unabhängig voneinander bis zu 10 C-Atome auf.

In bevorzugten Ausführungsformen ist R^{x} Alkyl oder substituiertes Alkyl. Gegenstand der Erfindung ist auch ein Verfahren zur Herstellung von Verbindungen der Formel **A** umfassend den Schritt a und nachfolgende Umwandlung der funktionellen Gruppen: 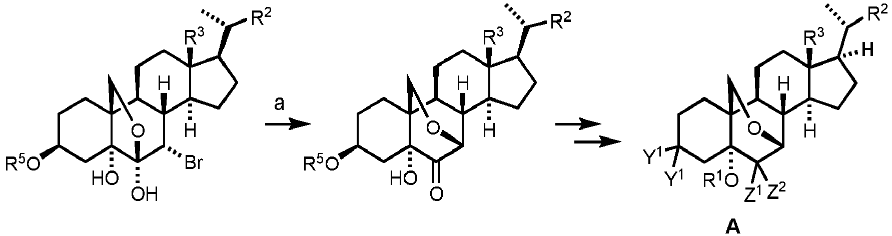 wobei Schritt a unter basischen Bedingungen, bevorzugt durch Erhitzen des Substrates in Gegenwart von Li₂CO₃ und LiBr in N,N-Dimethylformamid auf Temperaturen von 80 bis 120 °C durchgeführt wird.

Gegenstand der Erfindung ist auch die Verwendung der erfindungsgemäßen Arzneimittel in der Therapie maligner Erkrankungen, gutartigen bzw. semimalignen Hauterkrankungen.

Typische maligne Erkrankungen sind Erkrankungen, die das Knochenmark oder andere blutbildende Organe betreffen oder solide Tumore oder epitheliale Tumore sind, wobei die soliden Tumore insbesondere Hirntumore wie Glioblastom und Medulloblastom sind.

Typische gutartige bzw. semimaligne Erkrankungen sind Hauterkrankungen wie Psoriasis vulgaris, Keloide oder Basaliome.

Die erfindungsgemäßen Arzneimittel eignen sich auch zur Verwendung in der Therapie von entzündlichen und chronisch entzündlichen Erkrankungen, zur Anwendung zur immunsuppressiven Therapie, zur antiviralen, antibakteriellen, antimykotischen, anti-Protozoen- oder antihelminthischen Therapie.

Die Verbindungen werden bevorzugt intravenös im Konzentrationsbereich zwischen 0,1 bis 100 µg/ml, bezogen auf das Blutvolumen des Patienten, verabreicht.

Bei einer topischen Anwendung werden 0,1 bis 5 Gew.-%, bezogen auf das fertige Präparat, in die erkrankte Haut eingerieben.

Wie in den folgenden Beispielen gezeigt wird, zeichnen sich die erfindungsgemäßen Verbindungen durch folgende Eigenschaften aus:

### Wirkungsunterschiede zu herkömmlichen Glukokortikoiden

| | **Verbindungen Formel A** | **Glucocorticoide** |
|---|---|---|
| **Induktion von Nekrose (LDH)** | Nein | Nein |
| **Proliferationsinhibition** | +++ | + |
| **Zytostatika-Resistenzüberwindungen** | 8/8 | 0/8 |
| **Involvierung des intrinsischen Apoptosesignalweg** | Ja | Ja |
| **Involvierung des extrinsischen Apoptosesignalweg** | Ja | Ja? |
| **ROS-Induktion** | Nein | Ja |
| **ER-Stress-Induktion** | Ja | Ja (protektiv) |
| **Autophagie** | Ja (protektiv) | Ja (protektiv) |

Die neuartige Gruppe der 7,19-epoxy-Steroide zeigt im Gegensatz zu herkömmlichen Steroiden eine zytostatische Wirkung in soliden Tumorzellen, in verschiedenen Hirntumorzellen und in Steroid-resistenten Leukämie- und Lymphomzellen, die Ko-Resistenzen zu verschiedensten herkömmlichen in der Therapie verwendeten Zytostatika tragen. Ebenfalls ist eine Wirkung in Zytostatika-resistenten soliden Tumorzellen nachweisbar.

Die neuartige Steroid-Klasse induziert spezifisch Apoptose in malignen Zellen, während primäre humane Leukozyten nicht geschädigt werden.

Die neuartigen Steroide zeigen *in vitro* eine synergistische Wirkung mit herkömmlichen in der Therapie von malignen Erkrankungen verwendeten Zytostatika.

Die Zytostatika-Resistenz-Überwindung in Verbindung mit dem großen Verteilungsvolumen der Wirkstoffe im menschlichen Körper begründet durch ihre Steroid-Struktur sind die besonderen neuen Eigenschaften dieser neuen Wirkstoffklasse, die eine gute zytostatische Wirkung bei heutzutage schwer oder nicht therapierbaren malignen Erkrankungen versprechen lassen.

### Figuren

Figur 1 zeigt die Proliferationsinhibition in Nalm-6-Zellen.
Figur 2 zeigt die Steroid- und Polyzytostatika-Resistenzüberwindung in NaKu-Zellen.
Figur 3 zeigt die Polyzytostatika-Resistenzüberwindung in BiBo-Zellen.
Figur 4 zeigt die Polyzytostatika-Resistenzüberwindung in 7-CCA-Zellen.
Figur 5 zeigt synergistische Effekte mit Cytarabin und Vincristin auf Nalm-6-Zellen.
Figur 6 zeigt den Ausschluss von unspezifischer Nekrose bei Nalm-6-Zellen.
Figur 7 zeigt die Selektivität für Leukämiezellen (Nalm-6-Zellen) und in Lymphomzellen (BJAB-Zellen) im Vergleich zu humanen Leukozyten.
Figur 8 zeigt die Wirkung in Zytostatika-resistenten soliden Tumoren.
Figur 9 zeigt die Wirkung in Hirntumor-Zellen.
Figur 10 zeigt die Wirkung in primären humanen Glioblastom-Zellen.

### Beispiele

Die Erfindung wird durch die folgenden Beispiele näher erläutert.

### Beispiel 1: Synthese von 3β,5α-Dihydroxy-7β,19-epoxy-cholestan-6-on (9, WIL-071)

Die Synthesesequenz zur Herstellung der erfindungsgemäßen neuartigen Steroide wird am Beispiel der Substanz **WIL-071** erläutert. Dabei wird zunächst gemäß eines im Grundsatz literaturbekannten Verfahrens die C-19 Methylgruppe von Cholesterylacetat (**1**) hydroxyliert, bevor das 19-Hydroxycholesteryacatat (**4**) im B-Ring oxidativ funktionalisiert und die 7,19-Epoxybrücke geschlossen wird.

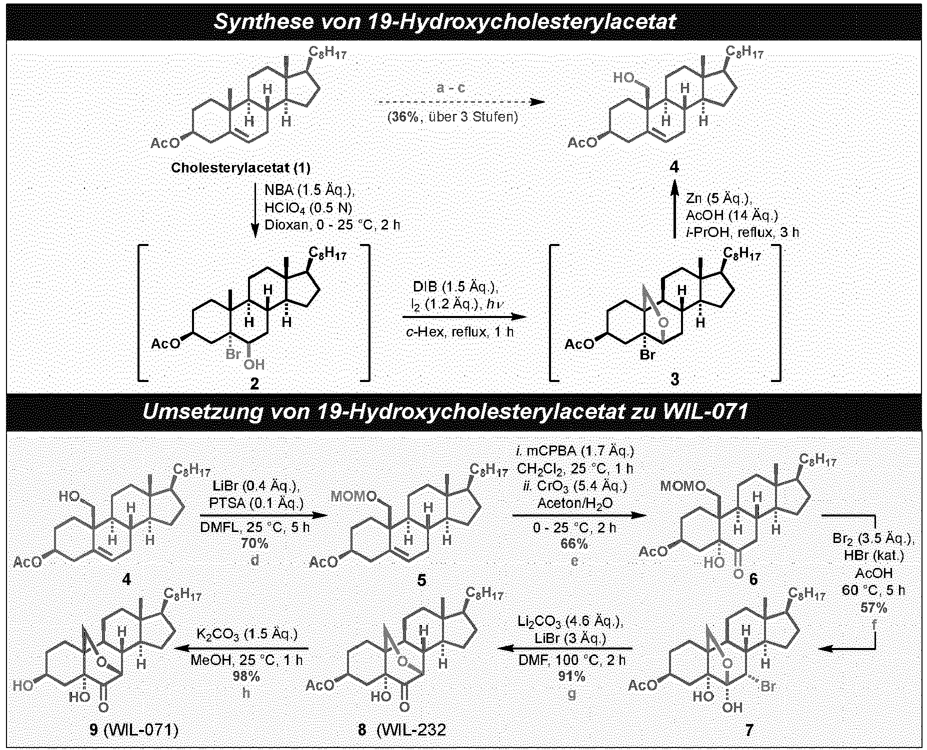

### Schritte a - c: Synthese von 3β-Acetoxy-cholest-Δ⁵-en-19β-ol (4)

200 g (0.23 mol, 1.0 Äq.) Cholesterylacetat (**1**) in 1.6 L Dioxan wurden mit 96 g (0.67 mol, 1.5 Äq.) *N*-Bromacetamid (NBA) sowie 320 mL HClO₄ (0.5 N) versetzt. Nach 1 h bei 0 °C unter Lichtausschluss und 1 h bei Raumtemperatur wurde die Reaktion durch Zugabe von gesättigter Na₂SO₃-Lösung (bis zur Verfärbung der Reaktionsmischung) und Wasser gestoppt. Nach Extraktion mit MTBE wurden die vereinigten organischen Phasen mit gesättigter NaCl-Lösung gewaschen und über MgSO₄ getrocknet. Anschließend wurde das Lösungsmittel unter vermindertem Druck entfernt. Das Rohprodukt (**2**, beiger Feststoff) - mit erheblichen Mengen (>40%) an regioisomerem Bromhydrin - wurde ohne weitere Aufreinigung im folgenden Schritt eingesetzt. Für eine detaillierte Analyse wurde eine kleine Menge des Bromhydrins **2** durch Säulenchromatographie aufgereinigt.

Zu einer Lösung von (ca. 25 g) Bromhydrin **2** (+ Regioisomer) in 1.5 L Cyclohexan wurden 27.6 g (85.8 mmol, 1.5 Äq.) Diacetoxyiodbenzol (DIB) sowie 8.76 g (68.6 mmol, 1.2 Äq.) Iod gegeben. Die Reaktionsmischung wurde für 1 h unter Bestrahlung mittels einer 150 Watt Quecksilberdampflampe zum Rückfluss erhitzt. Die violette Reaktionsmischung wurde auf Raumtemperatur gekühlt und durch Zugabe von gesättigter Na₂SO₃-Lösung und Wasser (bis zur Verfärbung) gequencht. Nach Extraktion mit EtOAc wurden die vereinigten organischen Phasen mit gesättigter NaCl-Lösung gewaschen und über MgSO₄ getrocknet. Anschließend wurde das Lösungsmittel unter vermindertem Druck entfernt und das Rohprodukt (**3**, braunes viskoses Öl) konnte im nächsten Schritt ohne weitere Aufreinigung eingesetzt werden. Diese Prozedur wurde insgesamt siebenfach vorgenommen. Eine kleine Menge an **3** wurde für analytische Zwecke säulenchromatographisch aufgereinigt.

Zu einer Lösung von Verbindung **3** (ca. 50 g) in 1.6 L *i*-PrOH wurden 37.8 g (0.58 mol, 5.0 Äq.) Zinkpulver sowie 94 mL (98 g, 1.64 mol, 14.0 Äq.) Essigsäure gegeben. Die Reaktionsmischung wurde für 3 h zum Rückfluss erhitzt und anschließend auf Raumtemperatur gekühlt und über Celite^{®} filtriert. Die klare, gelbe Lösung wurde auf ein Volumen von 100 mL eingeengt und nach Zugabe von Wasser mit MTBE extrahiert. Die vereinigten organischen Phasen wurden mit gesättigter NaCl-Lösung gewaschen und über MgSO₄ getrocknet. Das Lösungsmittel wurde unter vermindertem Druck entfernt. Diese Prozedur wurde insgesamt vierfach vorgenommen. Das Produkt **4** wurde nach säulenchromatographischer Aufreinigung an Kieselgel (c-Hex/EtOAc, 10:1) in Form eines beigen Feststoffs erhalten werden (74 g, 0.17 mol, 36% über drei Stufen).
- **Schmelzpunkt:**: 112 °C - 114 °C.
- **¹H-NMR**: (500 MHz, CDCl₃): δ [ppm] = 5.76 (d, *J* = 5.0 Hz, 1H), 4.66 - 4.60 (m, 1H), 3.82 (d, *J* = 11.2 Hz, 1H), 3.61 (dd, *J* = 11.4, 9.1 Hz, 1H), 2.41 (ddd, *J* = 13.0, 4.9 Hz, 2.1 Hz, 1H), 2.28 - 2.23 (m, 1H), 2.02 (s, 3H), 1.95 (dt, *J* = 13.8, 3.4 Hz, 1H), 1.90 - 0.93 (m, 26H), 0.90 (d, *J* = 6.5 Hz, 3H), 0.85 (dd, *J* = 6.6, 2.2 Hz, 3H), 0.72 (s, 3H).
- **¹³C-NMR**: (125 MHz, CDCl₃): δ [ppm] = 170.6, 134.7, 128.4, 73.6, 62.9, 57.7, 56.2, 50.4, 42.7, 41.7, 40.1, 39.6, 38.4, 36.3, 35.9, 33.5, 33.2, 31.4, 28.4, 28.2, 28.2, 24.2, 24.0, 23.0, 22.7, 21.9, 21.5, 18.8, 12.4.
- **FT-IR (ATR):**: *v*[cm⁻¹] = 3507 (bw), 2944 (s), 2934 (s), 2870 (m), 1734 (m), 1713 (s), 1469 (m), 1444 (m), 1381 (m), 1370 (m), 1254 (s), 1245 (vs), 1029 (vs), 977 (m), 961 (m), 911 (w), 886 (w), 824 (w), 807 (w), 742 (w), 670 (w), 610 (w).
- **HR-MS:**: (ESI, 70 eV) = *m*/*z* berechnet für: C₂₉H₄₈O₃Na⁺[M+Na]⁺ 467.3496 u, gefunden: 467.3493 u.

### Schritt d: Synthese von 3β-Acetoxy-19-methoxymethyloxy-cholestan-Δ⁵-en (5)

42 g (78.7 mmol, 1.0 Äq.) 19-Hydroxycholesterylacetat (**4**) in 315 mL CH₂(OMe)₂ wurden mit 1.64 g (18.9 mmol, 0.2 Äq.) Lithiumbromid sowie 1.8 g (9.44 mmol, 0.1 Äq.) *p*-Toluolsulfonsäure (als Monohydrat) versetzt und die Reaktionsmischung für 5 h bei Raumtemperatur gerührt. Nachdem ein vollständiger Umsatz beobachtet wurde (DC-Kontrolle), wurde die Reaktion durch Zugabe von gesättigter NaCl-Lösung gestoppt und die wässrige Phase mit MTBE extrahiert. Die vereinigten organischen Phasen wurden mit einer gesättigten NaHCO₃-Lösung sowie einer NaCl-Lösung gewaschen und über MgSO₄ getrocknet. Das Produkt **5** konnte nach säulenchromatographischer Aufreinigung an Kieselgel (c-Hex/EtOAc, 5:1) in Form eines weißen Feststoffs erhalten werden (26.9 g, 55.0 mmol, 70%).
- **Schmelzpunkt:**: 84 °C - 85 °C.
- **¹H-NMR**: (500 MHz, CDCl₃): δ [ppm] = 5.60 (d, *J* = 5.4 Hz, 1H), 4.67 - 4.58 (m, 3H), 3.73 (d, *J* = 10.3 Hz, 1H), 3.48 (d, *J* = 10.3 Hz, 1H), 3.37 (s, 3H), 2.40 (ddd, *J* = 13.0, 5.2, 2.2 Hz, 1H), 2.33 (t, *J* = 12.2 Hz, 1H), 2.11 (dt, *J* = 13.8, 3.7 Hz, 1H), 2.03 (s, 3H), 2.02 - 0.96 (m, 25H), 0.91 (d, *J* = 6.5 Hz, 3H), 0.86 (dd, *J* = 6.6, 2.3 Hz, 6H), 0.70 (s, 3H).
- **¹³C-NMR**: (125 MHz, CDCl₃): δ [ppm] = 170.7, 135.8, 126.2, 97.0, 73.8, 69.1, 57.4, 56.2, 55.6, 50.5, 42.6, 40.5, 40.2, 39.7, 38.5, 36.3, 36.0, 33.4, 32.9, 31.7, 28.4, 28.2, 28.2, 24.3, 24.0, 23.0, 22.7, 22.0, 21.6, 18.8, 12.2.
- **FT-IR (ATR):**: *v*[cm⁻¹] = 3419 (br w), 2932 (m), 2868 (m), 1733 (m), 1498 (w), 1467 (w), 1444 (w), 1366 (m), 1242 (s), 1144 (m), 1112 (m), 1096 (m), 1046 (s), 1033 (s), 986 (m), 962 (m), 943 (m), 915 (m), 881 (w), 845 (w), 824 (w), 814 (w), 727 (w), 703 (w), 679 (w), 630 (w), 607 (m), 580 (w).

### Schritt e: Synthese von 3β-Acetoxy-5α-hydroxy-19-methoxymethyloxy-cholestan-6-on (6)

32.4 g (66.3 mmol, 1.0 Äq.) der Verbindung **5** wurden in 400 mL CH₂Cl₂ gelöst und mit 25.3 g (113 mmol, 1.7 Äq.) *m*CPBA (70%) versetzt. Die Reaktionsmischung wurde 1 h bei Raumtemperatur gerührt (mit Erreichen eines vollen Umsatzes wurde ein weißer Niederschlag beobachtet, ist jedoch nicht zwingend notwendig) und auf 0 °C gekühlt. Der entstandene weiße Niederschlag wurde durch Zugabe von 950 mL Aceton gelöst und die klare Lösung wurde mit einer Lösung aus 24 g (239 mmol, 3.6 Äq.) CrO₃ in 80 mL Wasser versetzt. Nach 10 Minuten auf 0 °C wurde die Reaktionsmischung auf Raumtemperatur gebracht und für 1 h gerührt. Anschließend wurde die zuvor beschriebene Prozedur mit 7.82 g (78.2 mmol, 1.18 Äq.) CrO₃ in 35 mL Wasser wiederholt. Nach 2 h wurde die Reaktionsmischung durch Zugabe von gesättigter NaHCO₃-Lösung beendet und die wässrige Phase mit EtOAc extrahiert. Die vereinigten organischen Phasen wurden mit Wasser, gesättigter NaHCO₃-Lösung und gesättigter NaCl-Lösung gewaschen. Die klare, gelbe Lösung wurde über MgSO₄ getrocknet und das Lösungsmittel unter vermindertem Druck entfernt. Anschließend wurde das gewünschte Produkt **6** nach säulenchromatographischer Aufreinigung an Kieselgel (*c*-Hex/EtOAc, 8:1) in Form eines weißen Schaums erhalten (22.3 g, 42.8 mmol, 65%).
- **Schmelzpunkt:**: 143 °C - 144 °C.
- **¹H-NMR**: (500 MHz, CDCl₃): δ [ppm] = 5.11 (m, 1H), 4.50 (s, 2H), 3.64 (d, *J* = 10.6 Hz, 1H), 3.57 (d, *J* = 10.6 Hz, 1H), 3.34 (s, 3H), 2.75 (s, 1H), 2.57 (dd, *J* = 14.6, 11.6 Hz, 1H), 2.20 - 2.13 (m, 2H), 2.09 - 2.03 (m, 2H), 2.02 (s, 3H), 1.96 - 1.82 (m, 3H), 1.76 - 1.63 (m, 3H, H-1), 1.59 - 1.43 (m, 5H), 1.39 - 0.96 (m, 12H), 0.91 (d, *J* = 6.5 Hz, 3H), 0.86 (dd, *J* = 6.6, 2.1 Hz, 6H), 0.68 (s, 3H).
- **¹³C-NMR**: (125 MHz, CDCl₃): δ [ppm] = 209.6, 171.1, 97.1, 77.8, 70.4, 67.1, 57.0, 56.3, 56.1, 45.7, 44.1, 43.2, 41.5, 40.0, 39.6, 37.2, 36.3, 35.9, 32.5, 28.3, 28.1, 26.6, 26.1, 24.0, 22.9, 22.7, 21.9, 21.5, 18.8, 12.3.
- **FT-IR (ATR):**: *v*[cm⁻¹] = 3384 (br w), 2939 (m), 2869 (m), 1730 (m), 1713 (s), 1467 (w), 1401 (w), 1382 (m), 1365 (m), 1236 (s), 1150 (m), 1106 (m), 1034 (s), 1012 (s), 967 (m), 940 (m), 920 (m), 904 (m), 871 (w), 834 (w), 734 (w), 664 (w), 941 (w), 609 (w), 553 (w).

### Schritt f: Synthese von 3β-Acetoxy-7α-bromo-5α,6α-dihydroxy-6β,19-epoxy-cholestan (7)

Zu einer Lösung von 22.3 g (42.8 mmol, 1.0 Äq.) der Verbindung **6** in 400 mL Essigsäure wurden 6.6 mL (20.5 g, 128 mmol, 3.5 Äq.) Brom in Essigsäure (65 mL) sowie einige Tropfen HBr (48% wässr.) gegeben. Die Reaktionsmischung wurde für 5 h auf 60 °C erwärmt. Anschließend wurde die Reaktionsmischung auf Raumtemperatur abgekühlt und mit gesättigter Na₂SO₃-Lösung gequencht. Die wässrige Phase wurde mit EtOAc extrahiert, die vereinigten organischen Phasen wurden mit Wasser sowie gesättigter NaCl-Lösung gewaschen und über MgSO₄ getrocknet. Dann wurde das Lösungsmittel unter vermindertem Druck entfernt und das Produkt **7** wurde nach säulenchromatographischer Aufreinigung (c-Hex/EtOAc, 6:1) in Form eines weißen Feststoffs erhalten (13.4 g, 24.2 mmol, 57%).
- **Schmelzpunkt:**: 124 °C - 125 °C.
- **¹H-NMR**: (500 MHz, CDCl₃): δ [ppm] = 5.05 - 5.00 (m, 1H), 4.12 (d, *J* = 4.9 Hz, 1H), 4.02 (d, *J* = 9.0 Hz, 1H), 3.73 (d, *J* = 9.0 Hz, 1H), 3.09 (s, 1H), 2.89 (s, 1H), 2.19 (ddd, *J* = 12.9, 4.5, 2.2 Hz, 1H), 2.04 (s, 3H), 2.05 - 1.99 (m, 1H), 1.97 - 1.83 (m, 5H), 1.74 (t, *J* = 12.4 Hz, 1H), 1.62 (s, 2H), 1.59 - 0.98 (m, 19H), 0.91 (d, *J* = 6.5 Hz, 3H), 0.87 (dd, *J* = 6.6, 3.0 Hz, 6H), 0.74 (s, 3H).
- **¹³C-NMR**: (125 MHz, CDCl₃): δ [ppm] = 170.7, 101.7, 79.4, 69.9, 66.8, 59.4, 55.7, 52.9, 45.4, 43.5, 39.6, 39.0, 38.7, 38.6, 36.2, 35.8, 35.0, 28.2, 28.1, 27.2, 24.0, 24.0, 23.3, 23.0, 22.7, 21.6, 21.5, 18.8, 13.1.
- **FT-IR (ATR):**: *v* [cm⁻¹] = 3418 (br w), 2933 (m), 2868 (m), 1726 (s), 1714 (s), 1498 (w), 1466 (w), 1382 (m), 1366 (m), 1244 (s), 1154 (m), 1131 (m), 1096 (m), 1042 (s), 984 (m), 965 (s), 942 (s), 906 (m), 846 (m), 814 (w), 727 (w), 703 (m), 680 (m), 666 (m), 629 (m), 609 (m), 583 (m).

### Schritt g: Synthese von 3β-Acetoxy-5α-hydroxy-7β,19-epoxy-cholestan-6-one (8) (WIL-232)

22.6 g (40.7 mmol, 1.0 Äq.) der Verbindung **7** wurden in 1.8 L Dimethylformamid gelöst und mit 38.3 g (0.52 mol, 12.7 Äq.) Li₂CO₃ sowie 16.3 g (0.19 mol, 4.6 Äq.) LiBr versetzt. Die Reaktionsmischung wurde 2 h bei 100 °C gerührt. Anschließend wurde die Reaktionsmischung auf Raumtemperatur gekühlt und mit Wasser verdünnt. Die wässrige Phase wurde mit EtOAc extrahiert, die vereinigten organischen Phasen wurden mit Wasser sowie gesättigter NaCl-Lösung gewaschen und über MgSO₄ getrocknet. Anschließend wurde das Lösungsmittel unter vermindertem Druck entfernt und das Produkt **8** nach säulenchromatographischer Aufreinigung (*c*-Hex/EtOAc, 6:1) in Form eines weißen Feststoffs erhalten (17.5 g, 37.0 mmol, 91%).
- **Schmelzpunkt:**: 146 °C - 147 °C.
- **¹H-NMR**: (500 MHz, CDCl₃): δ [ppm] = 5.25 - 5.18 (m, 1H), 4.14 (dd, *J* = 10.2, 1.7 Hz, 1H), 3.91 (d, *J* = 10.2 Hz, 1H), 3.82 (d, *J* = 1.7 Hz, 1H), 2.61 (s, 1H), 2.12 - 2.03 (m, 3H), 2.03 (s, 3H), 2.00 - 1.94 (m, 2H), 1.86 (dt, *J* = 13.0, 9.4, 5.8 Hz, 1H), 1.73 (td, *J* = 13.8, 4.7 Hz, 1H), 1.65 - 1.48 (m, 5H), 1.42 - 1.04 (m, 13H), 0.98 (dt, *J* = 10.2, 8.7 Hz, 1H), 0.89 (d, *J* = 6.5 Hz, 3H), 0.86 (dd, *J* = 6.6, 2.4 Hz, 6H), 0.74 (s, 3H).
- **¹³C-NMR**: (125 MHz, CDCl₃): δ [ppm] = 212.9, 170.5, 78.7, 76.2, 69.2, 63.6, 55.7, 52.0, 45.5, 45.1, 43.2, 40.5, 40.2, 39.6, 36.2, 35.8, 35.2, 28.6, 28.1, 26.2, 23.9, 22.9, 22.9, 22.7, 21.7, 21.5, 21.3, 18.7, 13.1.
- **FT-IR (ATR):**: *v* [cm⁻¹] = 3507 (br w), 2944 (s), 2934 (s), 2870 (m), 1734 (m), 1713 (s), 1469 (m), 1444 (m), 1381 (m), 1370 (m), 1254 (s), 1245 (vs), 1029 (vs), 977 (m), 961 (m), 911 (w), 886 (w), 824 (w), 807 (w), 742 (w), 670 (w), 610 (w).
- **HR-MS:**: (ESI, 70 eV) = *m*/*z* berechnet für: C₂₉H₄₆O₅Na⁺ [M+Na]⁺ 497.32375 u, gefunden: 497.32341 u.

### Schritt h: Synthese von 3β,5α-Dihydroxy-7β,19-epoxy-cholestan-6-on (9) (WIL-071)

Zu einer Lösung von 12.7 g (26.7 mmol, 1.0 Äq.) **8** in 890 mL Methanol wurden 5.5 g (40.1 mmol, 1.5 Äq.) K₂CO₃ gegeben und die Reaktionsmischung für 1 h bei Raumtemperatur gerührt. Nach Abschluss der Reaktion (DC) wurde die Reaktionsmischung mit Wasser verdünnt. Die wässrige Phase wurde mit EtOAc extrahiert, die vereinigten organischen Phasen wurden mit NaCl-Lösung gewaschen und über MgSO₄ getrocknet. Anschließend wurde das Lösungsmittel unter vermindertem Druck entfernt und der Rückstand säulenchromatographisch (c-Hex/EtOAc, 1:1) aufgereinigt. Das Produkt **9** (WIL-071) wurde in Form eines weißen Feststoffs erhalten (11.3 g, 26.1 mmol, 98%) und anschließend aus ca. 400 mL (EtOH/H₂O, 1:1) umkristallisiert (9.7 g hochreine Substanz).
- **Schmelzpunkt:**: 178 °C - 180 °C.
- **¹H-NMR**: (600 MHz, CDCl₃): δ [ppm] = 4.19 - 4.13 (m, 2H), 3.88 (d, *J* = 10.2 Hz, 1H), 3.81 (s, 1H), 2.71 (s, 1H), 2.11 - 2.00 (m, 3H), 1.96 - 1.82 (m, 3H), 1.75 (s, 1H), 1.65 - 0.94 (m, 20H), 0.88 (d, *J* = 6.5 Hz, 3H), 0.85 (dd, *J* = 6.6, 2.9 Hz, 6H), 0.73 (s, 3H).
- **¹³C-NMR**: (150 MHz, CDCl₃): δ [ppm] = 213.6, 79.2, 76.2, 66.2, 63.7, 55.7, 52.1, 45.5, 45.2, 43.3, 40.6, 40.3, 39.6, 38.8, 36.2, 35.8, 30.2, 28.6, 28.1, 23.9, 22.9, 22.9, 22.7, 21.8, 21.5, 18.7, 13.1.
- **FT-IR (ATR):**: *v* [cm⁻¹] = 3463 (br w), 2948 (s), 2868 (m), 1733 (vs), 1497 (w), 1466 (m), 1414 (w), 1382 (m), 1365 (w), 1340 (w), 1296 (w), 1248 (m), 1225 (w), 1153 (m), 1051 (vs), 968 (m), 958 (m), 887 (w), 818 (w), 779 (w), 756 (m), 713 (w), 637 (w), 558 (m), 539 (w).
- **HR-MS:**: (ESI, 70 eV) = *m*/*z* berechnet für: C₂₇H₄₄O₄Na⁺ [M+Na]⁺ 455.31318 u, gefunden: 455.31365 u.

### Synthese von 5α-Hydroxy-7β,19-epoxy-cholestan-3,6-dion (10) (WIL-257)

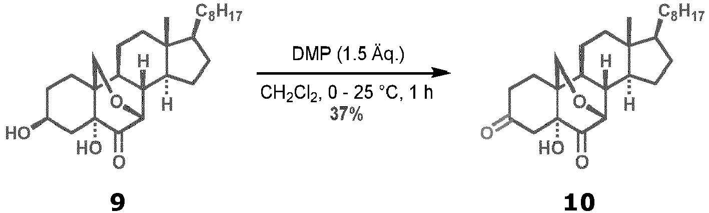

Zu einer Lösung von 400 mg (0.93 mmol, 1.0 Äq.) Alkohol **9** in 11.5 mL Dichlormethan bei 0 °C wurden 588 mg (1.39 mmol, 1.5 Äq.) *Dess-Martin-*Periodinan gegeben und die Reaktionsmischung wurde für 1 h bei Raumtemperatur gerührt. Nach Abschluss der Reaktion (DC) wurde die Reaktionsmischung mit wässriger NaHCO₃-Lösung und wässriger Na₂S₂O₃-Lösung verdünnt. Die wässrige Phase wurde mit CH₂Cl₂ extrahiert, die vereinigten organischen Phasen wurden über MgSO₄ getrocknet und das Lösungsmittel wurde unter vermindertem Druck entfernt. Der Rückstand wurde säulenchromatographisch (cHex/EtOAc, 5:1) aufgereinigt und das Produkt **10** in Form eines weißen Feststoffs erhalten (148 mg, 0.34 mmol, 37%).
- **Schmelzpunkt:**: 204 °C - 205 °C.
- **¹H-NMR**: (500 MHz, CDCl₃): δ [ppm] = 4.12 (q, J = 10.5 Hz, 2H), 3.93 (d, J = 1.6 Hz, 1H), 2.99 (d, J = 15.6 Hz, 1H), 2.92 - 2.87 (m, 1H), 2.48 (m, J = 16.3, 8.0 Hz, 2H), 2.43 - 2.34 (m, 1H), 2.19 (t, J = 10.6 Hz, 1H), 2.11 - 2.00 (m, 2H), 1.93 - 1.84 (m, 1H), 1.65 - 1.56 (m, 3H), 1.55 - 1.06 (m, 14H), 1.03 - 0.95 (m, 1H), 0.90 (d, J = 6.5 Hz, 3H), 0.86 (dd, J = 6.6, 2.5 Hz, 6H), 0.77 (s, 3H).
- **¹³C-NMR**: (126 MHz, CDCl₃): δ [ppm] = 211.5, 206.5, 80.0, 76.8, 63.6, 55.7, 51.9, 47.1, 45.4, 45.1, 43.4, 40.9, 40.2, 39.6, 36.9, 36.2, 35.8, 28.6, 28.1, 23.9, 22.9, 22.9, 22.7, 22.4, 21.8, 18.7, 13.1.
- **FT-IR (ATR):**: *v* [cm⁻¹] = 3459 (bw), 2952 (m), 2940 (m), 2918 (m), 2859 (m), 1742 (s), 1714 (s), 1466 (m), 1382 (m), 1345 (w), 1298 (w), 1252 (w), 1238 (m), 1195 (w), 1163 (m), 1145 (m), 1102 (w), 1079 (w), 1051 (s), 980 (w), 948 (m), 913 (w), 903 (w), 891 (w), 854 (w), 822 (w), 797 (w), 752 (m), 723 (m), 663 (w), 631 (m), 594 (w), 559 (m), 525 (m).
- **HR-MS:**: (ESI, 70 eV) = *m*/*z* berechnet für: C₂₇H₄₃O₄⁺ [M+H]⁺ 431.31559 u, gefunden: 431.31544 u; *m*/*z* berechnet für: C₂₇H₄₂O₄Na⁺ [M+Na]⁺ 453.29753 u, gefunden: 453.29761 u.

### Synthese von 7β,19-epoxy-cholest-4-en-3,6-dion (11) (WIL-270)

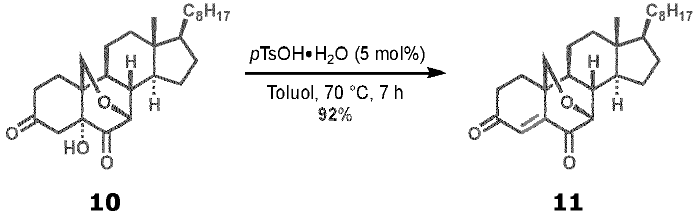

Zu einer Lösung von 50 mg (0.12 mmol, 1.0 Äq.) **10** in 1.2 mL Toluol wurden 1.8 mg (5.8 µmol, 0.05 Äq.) *p*TsOH·H₂O bei Raumtemperatur zugegeben. Das Reaktionsgemisch wurde auf 70 °C erwärmt und 7 h bei 70 °C gerührt. Nach Abschluss der Reaktion (DC) wurde die Reaktionsmischung mit CH₂Cl₂ und verdünnter wässriger NaHCO₃-Lösung verdünnt. Die wässrige Phase wurde mit CH₂Cl₂ extrahiert, die vereinigten org. Phasen wurde über MgSO₄ getrocknet und das Lösungsmittel wurde unter vermindertem Druck entfernt. Der Rückstand wurde säulenchromatographisch (cHex/EtOAc, 6:1) aufgereinigt. Das Produkt **11** wurde in Form eines weißen Feststoffs erhalten (44 mg, 0.11 mmol, 92%).
- **Schmelzpunkt:**: 127 °C - 128 °C.
- **¹H-NMR**: (500 MHz, CDCl₃): δ [ppm] = 6.42 (s, 1H), 4.03 (d, J = 8.9 Hz, 1H), 3.95 (s, 1H), 3.91 (d, J = 8.9 Hz, 1H), 2.50 - 2.43 (m, 2H), 2.23 (t, J = 10.9 Hz, 1H), 2.13 (dt, J = 13.2, 3.1 Hz, 1H), 2.06 - 1.96 (m, 1H), 1.95 - 1.83 (m, 2H), 1.70 - 1.62 (m, 1H), 1.62 - 1.47 (m, 3H), 1.44 - 1.06 (m, 12H), 1.03 - 0.96 (m, 1H), 0.90 (d, J = 6.5 Hz, 3H), 0.86 (dd, J = 6.6, 2.4 Hz, 6H), 0.78 (s, 3H)
- **¹³C-NMR**: (126 MHz, CDCl₃): δ [ppm] = 198.6, 197.7, 156.4, 122.7, 77.0, 64.3, 55.7, 52.4, 49.3, 45.4, 43.5, 40.3, 39.6, 38.4, 36.2, 35.8, 34.7, 28.7, 28.1, 24.2, 23.9, 22.9, 22.9, 22.7, 21.9, 18.7, 13.1.
- **FT-IR (ATR):**: *v* [cm⁻¹] = 2954 (m), 2933 (m), 2857 (m), 1724 (m), 1688 (s), 1632 (w), 1465 (m), 1453 (m), 1383 (w), 1366 (w), 1328 (w), 1274 (w), 1245 (w), 1231 (m), 1185 (m), 1113 (w), 1074 (w), 1035 (s), 974 (w), 932 (w), 904 (s), 879 (w), 857 (w), 828 (m), 811 (w), 780 (m), 766 (w), 720 (w), 653 (w), 634 (w), 571 (w), 548 (m), 529 (m), 507 (w).
- **HR-MS:**: (ESI, 70 eV) = *m*/*z* berechnet für: C₂₇H₄₁O₃⁺ [M+H]⁺ 413.30502 u, gefunden: 413.30513 u; *m*/*z* berechnet für: C₂₇H₄₀O₃Na⁺ [M+Na]⁺ 435.28697 u, gefunden: 435.28720 u.

### Synthese von 3β,5α-Dibenzyloxy-7β,19-epoxy-cholestan-6-on (12) (WIL 242a) sowie von 5α-Benzyloxy-3β-hydroxy-7β,19-epoxy-cholestan-6-on (13) (WIL 242b)

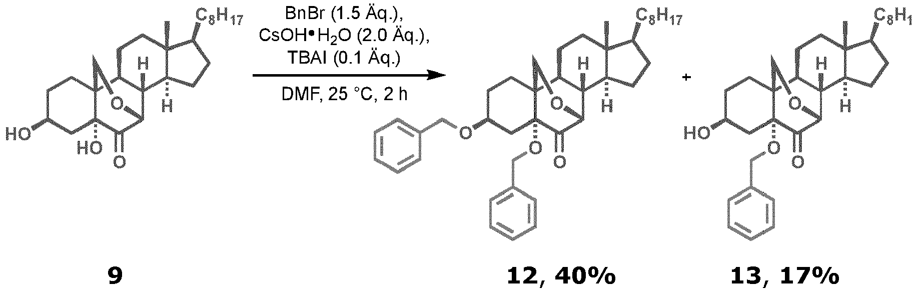

Zu einer Lösung von 39 mg (0.090 mmol, 1.0 Äq.) 9 in 0.92 mL Dimethylformamid wurden 35 mg (0.185 mmol, 2.0 Äq.) CsOH·H₂O, 16.2 mL (0.139 mmol, 1.5 Äq.) BnBr und 4 mg (0.011 mmol, 0.1 Äq.) Tetrabutylammoniumiodid bei Raumtemperatur zugegeben. Die Reaktionsmischung wurde 2 h bei Raumtemperatur gerührt. Nach Abschluss der Reaktion (DC) wurde die Reaktionsmischung mit MTBE und verdünnter wässriger NH₄Cl-Lösung verdünnt. Die wässrige Phase wurde mit MTBE extrahiert, die vereinigten org. Phasen wurden erst mit Wasser, dann mit gesättigter NaCl-Lösung gewaschen, anschließend über MgSO₄ getrocknet und das Lösungsmittel wurde unter vermindertem Druck entfernt. Der Rückstand wurde säulenchromatographisch (cHex/EtOAc, 9:1 auf 3:1) aufgereinigt. Das Produkt **12** (doppelt Bn) wurde in Form eines farblosen Öls erhalten (22 mg, 0.036 mmol, 40%). Zudem wurde das Produkt **13** (einfach benzyliert) ebenfalls in Form eines farblosen Öls erhalten (7.9 mg, 0.015 mmol, 17%).

Analytik zu 5*α*-Benzyloxy-3*β*-hydroxy-7*β*,19-epoxy-cholestan-6-on:
- **¹H-NMR**: (500 MHz, CDCl₃): δ [ppm] = 7.38 - 7.25 (m, 5H), 5.15 (d, *J* = 11.9 Hz, 1H), 4.79 (d, *J* = 12.0 Hz, 1H), 4.13 (d, *J* = 10.3 Hz, 1H), 3.88 (d, *J* = 10.2 Hz, 2H), 3.67 (s, 1H), 2.65 (dd, *J* = 14.2, 3.5 Hz, 1H), 2.09 - 1.97 (m, 3H), 1.96 - 1.73 (m, 4H), 1.63 - 1.06 (m, 18H), 1.02 - 0.95 (m, 1H), 0.89 (d, *J* = 6.5 Hz, 3H), 0.86 (dd, *J* = 6.6, 2.5 Hz, 6H), 0.74 (s, 3H).
- **¹³C-NMR**: (126 MHz, CDCl₃): δ [ppm] = 208.4, 139.3, 128.4, 127.1, 80.7, 66.1, 65.5, 63.7, 55.7, 52.6, 45.6, 44.1, 43.0, 41.4, 40.6, 39.6, 36.3, 35.8, 33.2, 30.5, 28.7, 28.2, 23.9, 23.0, 22.9, 22.7, 22.0, 21.9, 18.7, 13.1.
- **HR-MS:**: (ESI, 70 eV) = *m*/*z* berechnet für: C₃₄H₅₀O₄Na⁺ [M+Na]⁺ 545.36013 u, gefunden: 545.36016 u.

Analytik zu 3*β*,5*α*-Dibenzyloxy-7*β*,19-epoxy-cholestan-6-on:
- **¹H-NMR**: (500 MHz, CDCl₃): δ [ppm] = 7.38 - 7.23 (m, 8H), 7.16 (d, *J* = 6.9 Hz, 2H), 5.07 (d, *J* = 11.6 Hz, 1H), 4.58 - 4.51 (m, 3H), 4.15 (d, *J* = 9.9 Hz, 1H), 3.86 (d, *J* = 10.1 Hz, 1H), 3.66 (s, 1H), 3.64 - 3.55 (m, 1H), 2.72 (dd, *J* = 14.2, 3.4 Hz, 1H), 2.08 - 1.93 (m, 4H), 1.91 - 1.83 (m, 1H), 1.79 (dd, *J* = 14.1, 11.4 Hz, 1H), 1.72 (td, *J* = 13.8, 4.7 Hz, 1H), 1.62 - 1.46 (m, 4H), 1.41 - 1.06 (m, 13H), 1.02 - 0.94 (m, 1H), 0.89 (d, *J* = 6.4 Hz, 3H), 0.86 (dd, *J* = 6.6, 2.5 Hz, 6H), 0.73 (s, 3H).
- **¹³C-NMR**: (126 MHz, CDCl₃): δ [ppm] = 208.7, 139.2, 138.7, 128.6, 128.4, 127.8, 127.3, 127.3, 80.6, 72.3, 70.6, 65.5, 63.7, 55.8, 52.6, 45.6, 44.1, 43.1, 41.6, 40.6, 39.6, 36.3, 35.8, 30.2, 28.7, 28.2, 27.3, 23.9, 23.0, 23.0, 22.7, 21.9, 21.8, 18.7, 13.1, 0.2.
- **FT-IR (ATR):**: v [cm⁻¹] = 3064 (w), 3031 (w), 2949 (s), 2867 (s), 1733 (s), 1496 (w), 1454 (m), 1382 (w), 1364 (w), 1307 (w), 1262 (w), 1228 (w), 1206 (w), 1179 (w), 1142 (w), 1090 (s), 1054 (m), 1001 (w), 953 (w), 873 (w), 842 (w), 803 (w), 734 (s), 693 (s), 557 (w).
- **HR-MS:**: (ESI, 70 eV) = *m*/*z* berechnet für: C₄₁H₅₆O₄Na⁺ [M+Na]⁺ 635.40708 u, gefunden: 635.40751 u.

### Beispiel 2: Proliferationsinhibition in Leukämiezellen (Nalm-6-Zellen

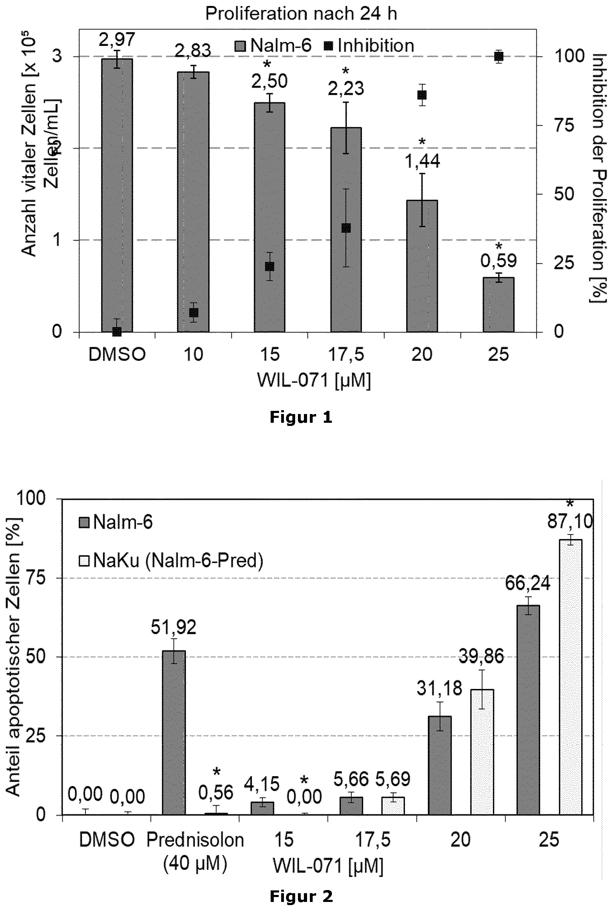

In Leukämiezellen (Nalm-6) wird die Zellproliferation gehemmt. 1 × 10⁵ Zellen vermehren sich nach 24 h auf fast das Dreifache (2.97 ×10⁵ Zellen), während die Zell-Proliferation durch Behandlung mit dem Steroid WIL 071 konzentrationsabhängig bis zu 100% gehemmt wird, siehe **Figur 1**. Die Experimente sind dreimal unabhängig voneinander durchgeführt worden. Die Fehlerbalken zeigen die Standardabweichungen der Messwerte von drei unabhängigen Experimenten.

### Beispiel 3: Steroid- und Polyzytostatika-Resistenzüberwindung in NaKu-Zellen

In Steroid- und Polyzytostatika-resistenten Leukämiezellen (NaKu (ALL)) wird durch Behandlung mit dem Steroid WIL 071 konzentrationsunabhängig eben so viel Apoptose induziert wie in den nicht resistenten Leukämiezellen (Nalm-6), siehe **Figur 2****.** Die Experimente sind dreimal unabhängig voneinander durchgeführt worden. Die Fehlerbalken zeigen die Standardabweichungen der Messwerte von drei unabhängigen Experimenten.

Das Steroid WIL 071 überwindet die Steroidresistenz in Leukämiezellen (NaKu-Zellen). Ferner weisen die Steroid-resistenten Leukämiezellen (NaKu-Zellen) Ko-Resistenzen für folgende Zytostatika auf:
- Prednisolon, Methylprednisolon, Dexamethason,
- Doxorubicin, Daunorubicin, Idarubicin, Epirubicin
- Cytarabin, Cladribin
- Etoposid
- Cyclophosphamid, Ifosfamid
- Vindesin
- Cisplatin, Carboplatin, Oxaliplatin

### Beispiel 4: Polyzytostatika-Resistenzüberwindung in BiBo-Zellen

BiBo-Zellen sind Lymphomzellen (BJAB-Zellen), die resistent gegen Vincristin gemacht worden sind und als Resistenzmechanismus eine Bcl-2 Überexpression aufweisen. Sie weisen Ko-Resistenzen für folgende Zytostatika auf:
- Vincristin, Vinblastin, Vindesin, Vinorelbin, Taxol
- Cytarabin
- Daunorubicin

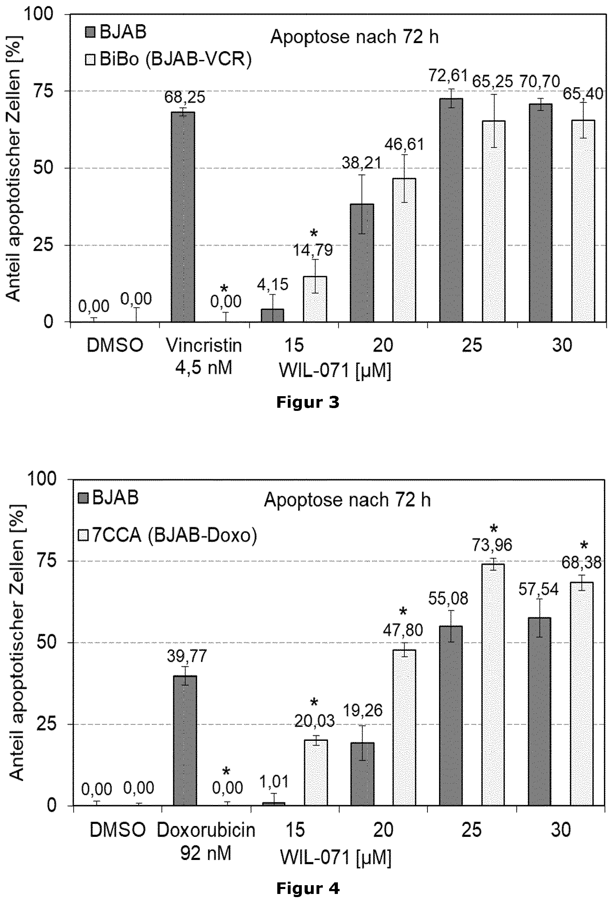

In Polyzytostatika-resistenten Lymphomzellen (BiBo-Zellen) wird durch Behandlung mit dem Steroid WIL 071 konzentrationsunabhängig eben so viel Apoptose induziert wie in den nicht resistenten Lymphomzellen (BJAB (Burkitt like lymphoma Zellen)), siehe **Figur 3****.** Die Experimente sind dreimal unabhängig voneinander durchgeführt worden. Die Fehlerbalken zeigen die Standardabweichungen der Messwerte von drei unabhängigen Experimenten. Das Steroid WIL 071 überwindet die Steroidresistenz in Lymphomzellen (BiBo-Zellen).

### Beispiel 5: Polyzytostatika-Resistenzüberwindung in 7-CCA-Zellen

7-CCA-Zellen sind Lymphomzellen (BJAB-Zellen), die resistent gegen Doxorubin gemacht worden sind und als Resistenzmechanismus eine Caspase-3-Unterexpression aufweisen. Sie weisen Ko-Resistenzen für folgende Zytostatika auf:
- Vincristin, Vinblastin, Vindesin, Vinorelbin, Taxol
- Cytarabin, Fudarabin, Cladribin, Clofarabin,
- Daunorubicin, Doxorubicin, Idarubicin, Epirubicin
- Cisplatin, Oxaliplatin

In Poly-Zytostatika-resistenten Lymphomzellen (7-CCA-Zellen) wird durch Behandlung mit dem Steroid WIL 071 konzentrationsunabhängig eben so viel Apoptose induziert wie in den nicht resistenten Lymphomzellen (BJAB (Burkitt like lymphoma Zellen)), siehe **Figur 4****.** Die Experimente sind dreimal unabhängig voneinander durchgeführt worden. Die Fehlerbalken zeigen die Standardabweichungen der Messwerte von drei unabhängigen Experimenten. Das Steroid WIL 071 überwindet die Steroidresistenz in Lymphomzellen (7-CCA-Zellen).

### Beispiel 6: Synergistische Effekte mit Cytarabin und Vincristin

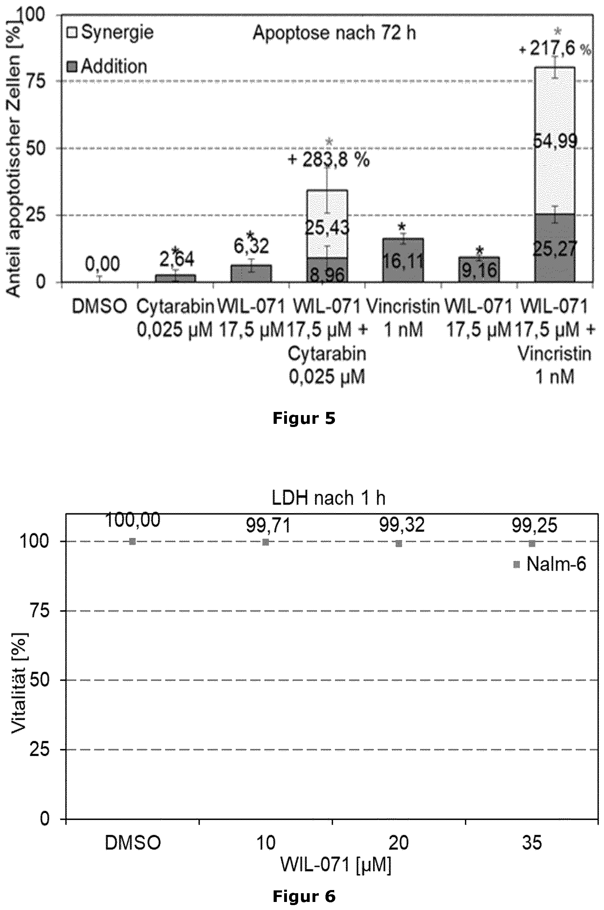

In Leukämiezellen (Nalm-6-Zellen) wird durch parallele Behandlung mit dem Steroid WIL 071 und Cytarabin bzw. Vincristin signifikant mehr Apoptose induziert als das 1.1 fache der Summe der Apoptose-Raten bei der Behandlung durch Monotherapie der entsprechenden Wirkstoffe, siehe **Figur 5****.** Die Experimente sind dreimal unabhängig voneinander durchgeführt worden. Die Fehlerbalken zeigen die Standardabweichungen der Messwerte von drei unabhängigen Experimenten. Das Steroid WIL 071 wirkt synergistisch mit Cytarabin bzw. Vincristin.

### Beispiel 7: Ausschluss von unspezifischer Nekrose

Gemessen ist die Lactatdehydrogenase (LDH)-Freisetzung in Leukämiezellen (Nalm-6-Zellen) im Zellüberstand, welche ein Zeichen des unspezifischen Zelltodes (Nekrose) ist, da dieses große Protein LDH nur eine defekte Zellmembran in Richtung extrazellulären Raum überschreiten kann, siehe **Figur 6****.** Unspezifische Nekrose kann ausgeschlossen werden.

### Beispiel 8: Selektivität

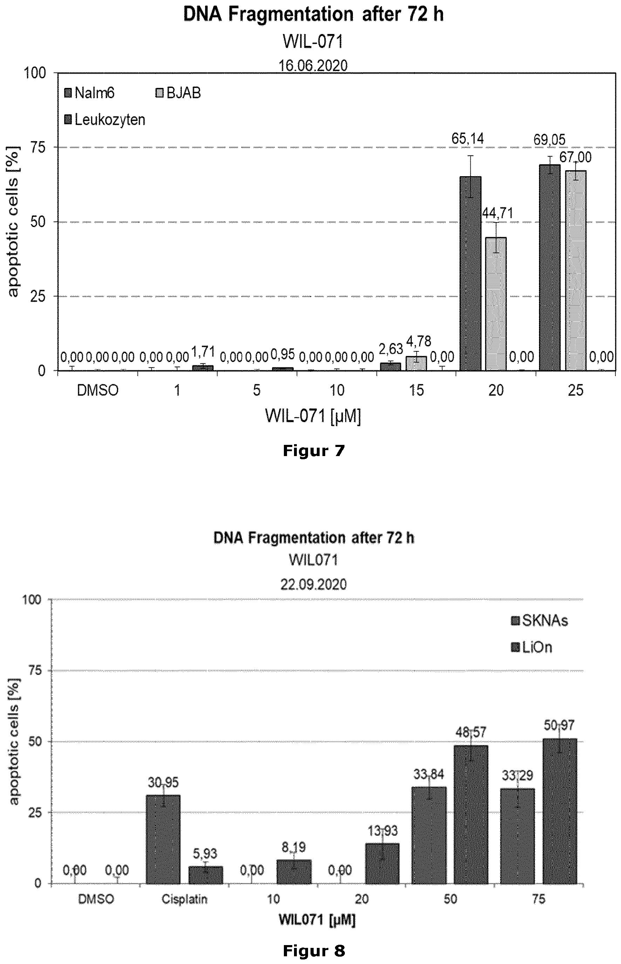

In Leukämiezellen (Nalm-6-Zellen) und in Lymphomzellen (BJAB-Zellen) wird durch Behandlung mit dem Steroid WIL 071 konzentrationsabhängig Apoptose induziert. WIL 071 wirkt selektiv in malignen Zellen, die proliferieren. In humanen primären Leukozyten wird dagegen keine Apoptose induziert. Durchflusszytometrisch wurde die DNA-Fragmentierung gemessen, siehe **Figur 7****.** Die Experimente sind dreimal unabhängig voneinander durchgeführt worden. Die Fehlerbalken zeigen die Standardabweichungen der Messwerte von drei unabhängigen Experimenten.

### Beispiel 9: Wirkung in Zytostatika-resistenten soliden Tumoren

Im Gegensatz zu herkömmlichen Glukokortikoiden kann das Steroid WIL 071 auch in soliden Tumorzellen Apoptose induzieren. Auch in Neuroblastomzellen (SKNAS-Zellen) werden Resistenzen gegenüber Zytostatika überwunden (LiOn-Zellen - Cisplatin-resistente SKNAS-Zellen mit einer Unterexpression von Caspase-8.

Durchflusszytometrisch wurde die DNA-Fragmentierung gemessen), siehe **Figur 8****.** Die Experimente sind dreimal unabhängig voneinander durchgeführt worden. Die Fehlerbalken zeigen die Standardabweichungen der Messwerte von drei unabhängigen Experimenten.

### Beispiel 10: Wirkung in Hirntumor-Zellen

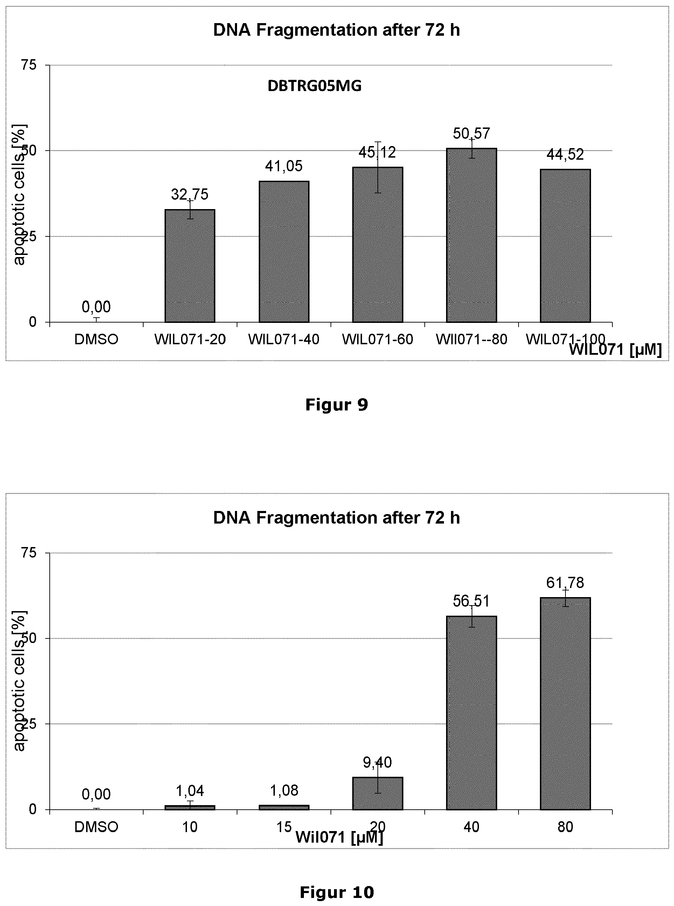

Im Gegensatz zu herkömmlichen Glukokortikoiden kann das Steroid WIL 071 auch in Hirntumorzellen Apoptose induzieren. Auch in Glioblastomzellen (DBTRG05MG-Zellen) induziert das Steroid WIL 071 signifikant Apoptose. Durchflusszytometrisch wurde die DNA-Fragmentierung gemessen, siehe **Figur 9****.** Die Experimente sind dreimal unabhängig voneinander durchgeführt worden. Die Fehlerbalken zeigen die Standardabweichungen der Messwerte von drei unabhängigen Experimenten.

### Beispiel 11: Wirkung in primären humanen Glioblastom-Zellen

Im Gegensatz zu herkömmlichen Glukokortikoiden kann das Steroid WIL 071 auch in primären humanen Hirntumorzellen Apoptose induzieren. Auch in primären Glioblastom-Zellen eines Patienten mit Glioblastom (Grad 4) induziert das Steroid WIL 071 signifikant Apoptose.

Durchflusszytometrisch wurde die DNA-Fragmentierung gemessen, siehe **Figur 10****.** Die Experimente sind dreimal unabhängig voneinander durchgeführt worden. Die Fehlerbalken zeigen die Standardabweichungen der Messwerte von drei unabhängigen Experimenten.

### Beispiel 12: Vergleich der biologischen Wirkung verschiedener Steroidderivate in Leukämie-Zellen (Nalm-6)

| **Steroid-Derivat** | **Biologische Wirkung:** halbmaximale Apoptoseinduktion (AC₅₀) [µM] |
|---|---|
| WIL-071 | 16 |
| WIL-232 | 24 |
| WIL-257 | 41 |
| WIL-242b | 43 |
| WIL-270 | 16 |

*Apoptoseinduktion in Nalm-6-Zellen nach der Behandlung mit WIL-071 bzw. verschiedenen Steroid-Derivaten.*

Die Zellen wurden in 6-Well-Platten mit 1,0·10⁵ Zellen/mL in zwei Millilitern Medium mit dem jeweiligen Steroid-Derivat in verschiedenen Konzentrationen inkubiert. Als Kontrolle diente die mitgeführte Lösungsmittelkontrolle (DMSO), die von allen weiteren gemessenen Werten subtrahiert wurde, um die unabhängig von der Wirkstoffkonzentration stattfindende Spontanapoptose der Zellen nicht zu berücksichtigen. Nach 72 h wurde eine modifizierte Zellzyklusanalyse durchgeführt, um den Anteil apoptotischer Zellen mittels Durchflusszytometrie zu quantifizieren. Es wurden für jede Konzentration des Steroids jeweils die Mittelwerte ± Standardabweichungen, n = 3 (^{∗}: p < 0,05 vs. DMSO, T-Test) ermittelt. Mit Hilfe der aus diesen Messwerten entstehenden Kurve der Dosis-Wirkungsbeziehung (Steroid-Konzentration-Apoptoseinduktion) wurde die Steroid-Konzentration abgelesen, bei welcher in 50% der getesteten Leukämie-Zellen Apoptose ausgelöst wurde.

### Literatur

### Apoptose:

G. M. Cohen (1997), Caspases: the executioners of apoptosis, Biochem. J. 326, 1-16.
F. Eßmann, T. Wieder, A. Otto, E.-C. Müller, B. Dörken, P. T. Daniel (2000), The GDP dissociation inhibitor, D4-GDI (Rho-GDI 2), but not the homologous Rho-GDI 1, is cleaved by caspase-3 during drug-induced apoptosis, Biochem. J. 346, 777-783.
Y. A. Hannun (1997), Apoptosis and the dilemma of cancer therapy, Blood 89, 1845-1853.
J. A. Hickman (1996), Apoptosis and chemotherapy resistance, Eur. J. Cancer 32A, 921-926.
M. Raisova, M. Bektas, T. Wieder, P. T. Daniel, J. Eberle, C. E. Orfanos, C. C. Geilen (2000), Resistance to CD95/Fas-induced and ceramide-mediated apoptosis of human melanoma cells is caused by a defective mitochondrial cytochrome c release, FEBS Lett. 473, 27-32.

### Physiologische Funktion der Glucocorticoide:

Munck A, Guyre PM, Holbrook NJ (1984). Physiological functions of glucocorticoids in stress and their relation to pharmacological actions. Endocr Rev, 5, 25-44.

### Glucocorticoide für die Therapie maligner Erkrankungen:

Keith BD (2008). Systematic review of the clinical effect of glucocorticoids on nonhematologic malignancy. BMC Cancer, 8, 84.

### Prednison good vs. poor responder:

Dördelmann M, Reiter A, Borkhardt A, Ludwig WD, Götz N, Viehmann S, Gadner H, Riehm H, Schrappe M (1999). Prednisone response is the strongest predictor of treatment outcome in infant acute lymphoblastic leukemia. Blood, 94, 1209-1217.
Pufall MA (2015). Glucocorticoids and Cancer. Adv Exp Med Biol, 872, 315-333.

### Wirkmechanismus:

### Allgemein:

Geley S, Fiegl M, Hartmann BL, Kofler R (1996). Genes mediating glucocorticoid effects and mechanisms of their regulation. Reviews of physiology, biochemistry and pharmacology, 128, 1-97.
Giguère V, Hollenberg SM, Rosenfeld MG, Evans RM (1986). Functional domains of the human glucocorticoid receptor. Cell, 46, 645-652.
Kuida K, Haydar TF, Kuan C-Y, Gu Y, Taya C, Karasuyama H, Su MS-S, Rakic P, Flavell RA (1998). Reduced Apoptosis and Cytochrome c-Mediated Caspase Activation in Mice Lacking Caspase 9. Cell, 94, 325-337.
Mattern J, Büchler MW, Herr I (2007). Cell cycle arrest by glucocorticoids may protect normal tissue and solid tumors from cancer therapy. Cancer Biol Ther, 6, 1345-1354.
Greenstein S, Ghias K, Krett NL, Rosen ST (2002). Mechanisms of glucocorticoid-mediated apoptosis in hematological malignancies. Clin Cancer Res, 8, 1681-1694.
Miyashita T, Nagao K, Krajewski S, Salvesen GS, Reed JC, Inoue T, Yamada M (1998). Investigation of glucocorticoid-induced apoptotic pathway: processing of caspase-6 but not caspase-3. Cell death and differentiation, 5, 1034-1041.
Planey SL, Abrams MT, Robertson NM, Litwack G (2003). Role of apical caspases and glucocorticoid-regulated genes in glucocorticoid-induced apoptosis of pre-B leukemic cells. Cancer Res, 63, 172-178.
Rathmell JC, Lindsten T, Zong W-X, Cinalli RM, Thompson CB (2002). Deficiency in Bak and Bax perturbs thymic selection and lymphoid homeostasis. Nat Immunol, 3, 932-939.
Almond JB, Snowden RT, Hunter A, Dinsdale D, Cain K, Cohen GM (2001). Proteasome inhibitor-induced apoptosis of B-chronic lymphocytic leukaemia cells involves cytochrome c release and caspase activation, accompanied by formation of an approximately 700 kDa Apaf-1 containing apoptosome complex. Leukemia, 15, 1388-1397.

### Wechselwirkungen mit Transkriptionfaktoren:

Herrlich P (2001). Cross-talk between glucocorticoid receptor and AP-1. Oncogene, 20, 2465-2475.

### Zellzyklusarrest:

Mattern J, Büchler MW, Herr I (2007). Cell cycle arrest by glucocorticoids may protect normal tissue and solid tumors from cancer therapy. Cancer Biol Ther, 6, 1345-1354.
Greenstein S, Ghias K, Krett NL, Rosen ST (2002). Mechanisms of glucocorticoid-mediated apoptosis in hematological malignancies. Clin Cancer Res, 8, 1681-1694.
Rogatsky I, Trowbridge JM, Garabedian MJ (1997). Glucocorticoid receptormediated cell cycle arrest is achieved through distinct cell-specific transcriptional regulatory mechanisms. Mol Cell Biol, 17, 3181-3193.

### ER-Stress, ROS, Autophagie:

Aoki S, Morita M, Hirao T, Yamaguchi M, Shiratori R, Kikuya M, Chibana H, Ito K (2017). Shift in energy metabolism caused by glucocorticoids enhances the effect of cytotoxic anti-cancer drugs against acute lymphoblastic leukemia cells. Oncotarget, 8, 94271-94285.
Liu W, Zhao Z, Na Y, Meng C, Wang J, Bai R (2018). Dexamethasone-induced production of reactive oxygen species promotes apoptosis via endoplasmic reticulum stress and autophagy in MC3T3-E1 cells. Int J Mol Med, 41, 2028-2036.
Yang J, Wu Q, Lv J, Nie H (2017). 4-Phenyl butyric acid prevents glucocorticoid-induced osteoblast apoptosis by attenuating endoplasmic reticulum stress. Journal of bone and mineral metabolism, 35, 366-374.

## Patentansprüche

1. Arzneimittel auf Basis von 7,19-epoxy-Steroiden, enthaltend eine Verbindung der allgemeinen Formel A 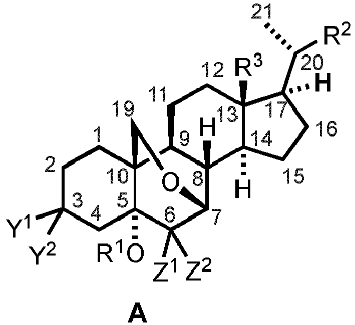 oder ein physiologisch verträgliches Salz hiervon, worin in der Formel bedeuten:
R¹ = H; Methyl, Aryl-CH₂-, R^{x}-C(=O)- oder R^{x}-O-C(=O)- ; oder R¹O entfällt, wenn eine Doppelbindung zwischen 4-5 vorhanden ist;
R² = gesättigte oder ungesättigte, gegebenenfalls verzweigte Alkylreste mit 4 bis 9 C-Atomen, wobei diese Alkylreste können auch eine Cyclopropan- oder Cyclobutan-Substruktur aufweisen;
R³= Methyl oder Ethyl,
Y¹, Y² = (H/OR⁵), oder zusammen O, NOR⁴;
Z¹, Z² = (H/OR⁵), oder zusammen O, NOR⁴;
R⁴ unabhängig voneinander = H, Alkyl oder Aryl; und
R⁵ unabhängig voneinander = H; Alkyl, Aryl-CH₂-, R^{x}-C(=O)- oder R^{x}-O-C(=O)-;
wobei jedes R^{x} unabhängig voneinander = H, gesättigte oder ungesättigte, gegebenenfalls verzweigtes Alkyl, gesättigte oder ungesättigte, gegebenenfalls verzweigtes substituiertes Alkyl, Aryl, Heteroaryl, PEG, SO₃H, PO(OH)₂ oder Glycosyl ist;
und wobei bis zu 3 Wasserstoffe durch Fluor substituiert sein können.

2. Arzneimittel nach Anspruch 1, wobei R¹ = H, Z¹, Z² = O, R³ = Methyl ist.

3. Arzneimittel nach Anspruch 1 oder 2, wobei Y¹, Y² = (H/OH) oder Y¹, Y² = (H/O R⁵), wobei R⁵ = R^{x}-C(=O)- oder R^{x}-O-C(=O)- und R^{x} = H, Alkyl, substituiertes Alkyl, Aryl, Heteroaryl ist.

4. Arzneimittel nach einem der vorherigen Ansprüche,
wobei R^{x} = H, Alkyl und R² 4-Methyl-pentyl ist.

5. Arzneimittel nach einem der vorherigen Ansprüche, wobei R¹ = H; Methyl.

6. Arzneimittel nach einem der vorherigen Ansprüche, wobei R⁴ bis zu 10 C-Atome aufweist.

7. Arzneimittel nach einem der vorherigen Ansprüche, wobei R⁵ bis zu 10 C-Atome aufweist.

8. Arzneimittel nach einem der vorherigen Ansprüche, wobei R^{x} bis zu 10 C-Atome aufweist.

9. Arzneimittel nach einem der vorherigen Ansprüche, wobei R^{x} Alkyl oder substituiertes Alkyl ist.

10. Verfahren zur Herstellung von Verbindungen der Formel **A** umfassend den Schritt a und nachfolgende Umwandlung der funktionellen Gruppen: 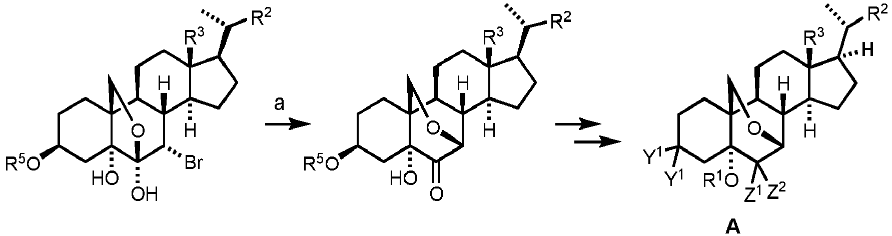 wobei Schritt a unter basischen Bedingungen durchgeführt wird.

11. Verbindung der allgemeinen Formel **A** wie im Anspruch 1 definiert zur Verwendung in der Therapie maligner Erkrankungen, gutartigen bzw. semimalignen Hauterkrankungen.

12. Verbindung zur Verwendung nach Anspruch 11, wobei die malignen Erkrankungen das Knochenmark oder andere blutbildender Organe betreffen oder solide Tumore oder epitheliale Tumore sind, insbesondere wobei die soliden Tumore Hirntumore (Grad 1 - Grad 4), insbesondere Glioblastom und Medulloblastom sind.

13. Verbindung zur Verwendung nach Anspruch 11, wobei die gutartigen bzw. semimalignen Hauterkrankungen Psoriasis vulgaris, Keloide oder Basaliome sind.

14. Verbindung der allgemeinen Formel **A** wie im Anspruch 1 definiert zur Verwendung in der Therapie von entzündlichen und chronisch entzündlichen Erkrankungen, zur Anwendung zur immunsuppressiven Therapie, zur antiviralen, antibakteriellen, antimykotischen, anti-Protozoen- oder antihelminthischen Therapie.

15. Verbindung zur Verwendung nach Anspruch 11, wobei die Therapie mit einem Zytostatikum kombiniert wird, insbesondere ausgewählt aus der Gruppe umfassend Cytarabin und Vincristin.
